# EUROPEAN PATENT APPLICATION

(11) **EP 4 696 341 A1**
(43) Date of publication of application: **18.02.2026**
(21) Application number: 24814120.2
(22) Date of filing: 07.05.2024
(51) Int. Cl.: A61L 29/08, A61L 29/16, A61M 25/10, A61F 2/82, A61F 2/88, A61F 2/90, A61F 2/86, A61M 31/00

(54) **MEDICAL DEVICE AND SYSTEM, AND PREPARATION METHOD**

(30) Priority: 29.05.2023 CN 202310620642
(71) Applicant: Shanghai MicroPort Medical (Group) Co., Ltd., Shanghai 201203 (CN)
(72) Inventor: ZHANG, Zhenghai, Shanghai 201203 (CN); WU, Jintian, Shanghai 201203 (CN); WANG, Jing, Shanghai 201203 (CN); ZHOU, Qi, Shanghai 201203 (CN)
(74) Representative: Marks & Clerk LLP
(86) International application number: PCT/CN2024/091407
(87) International publication number: WO 2024/244915

(57) **Abstract**

The present invention provides a medical device and system and a method of preparation. The medical device includes a radially expandable and collapsible base body and a coating layer disposed over at least portion of an outer surface of the base body. The coating layer contains a protein and a crosslinking agent. The base body may be a balloon body and can be introduced into a diseased blood vessel, and the protein experiences crosslinking reactions with proteins on the wall of the blood vessel in the presence of the crosslinking agent, forming an in-situ "protein micro-scaffold" to support the vessel wall, thereby maintaining the luminal gain and dilated size, and reducing the risk of restenosis.

## Description

### TECHNICAL FIELD

The present invention pertains to the technical field of medical device. In particular, it relates to a medical device and system and a method of preparation.

### BACKGROUND

Atherosclerosis, which has been regarded as one of the major contributors of heart disease and stroke, is thickening and narrowing of arteries that supply blood to various organs, including the heart, brain and kidneys. There are a number of options available for the treatment of atherosclerosis, including balloon angioplasty, stent implantation and atherectomy, and the stent implantation is mostly used. However, as a long-term implant in the body, the stent implantation still faces risks of long-term restenosis and thrombosis.

The advent of degradable stents has addressed the risks associated with long-term implant in the body. However, as they are still foreign implants, patients need to continue taking medication in the early stage after implantation. Additionally, the degradation of synthetic materials that are used to make the degradable stent results in the acidification of the vascular wall matrix, potentially exacerbating inflammation and leading to calcification. As an alternative therapy not suffering from the problems with foreign implantation, the drug-coated balloon is used for one-time drug delivery simply by physically attaching a drug coating to a blood vessel wall. However, due to the absence of support from the blood vessel, drug loss can easily occur, making it difficult to maintain an adequate drug concentration for a sufficiently long time. Therefore, good therapeutic outcomes and luminal gain over a long period of time cannot be expected. As another alternative therapy, percutaneous transluminal angioplasty (PTA) is often used in peripheral arterial disease (PAD), but its benefits are subpar due to vascular recoil and dissection.

### SUMMARY OF THE INVENTION

It is an object of the present invention to provide a medical device and system and a method of preparation, which can provide better treatment of atherosclerosis.

To this end, the present invention provides a medical device comprising a base body and a coating layer. The base body is radially expandable and collapsible, and the coating layer is disposed over at least portion of an outer surface of the base body and comprises a protein and a crosslinking agent.

Optionally, the crosslinking agent may comprise a photosensitive crosslinker, when exposed to light, the photosensitive crosslinker undergoes a cross-linking reaction with the protein.

Optionally, the photosensitive crosslinker may comprise at least one of methylene blue, methylene green, rose Bengal, riboflavin, proflavin, fluorescein, eosin, 4-amino-1,8-naphthalimide, 2,2'-((ethane-1,2-diylbis(oxy))bis(ethane-2,1-diyl))bis(6-((2-(2-(2-aminoethoxy)ethoxy)ethyl)amino)-1H-benzo[de]isoquinoline -1,3(2H)-dione) and salts thereof.

Optionally, the crosslinking agent may comprise a non-photosensitive crosslinker, wherein the coating layer further comprises an isolator that isolates the protein from the non-photosensitive crosslinker, thereby preventing the protein from undergoing a crosslinking reaction with the non-photosensitive crosslinker.

Optionally, the non-photosensitive crosslinker may comprise a physical crosslinker and/or a chemical crosslinker, the chemical crosslinker comprising at least one of glutaraldehyde, polyepoxide, genipin, horseradish catalase, ammonium persulfate, tris(bipyridine)ruthenium(II) chloride, camphorquinone and curcumin.

Optionally, the physical crosslinker may comprise at least one of tannic acid, pentagalloylglucose, gallate, gallic acid, ellagic acid, proanthocyanidin, anthocyanin, quercetin, lignin (yellow), catechin and epicatechin.

Optionally, the protein may comprise at least one of humanized elastin, humanized collagen, human serum albumin, fibroin and proteoglycan.

Optionally, a weight ratio of the protein to the crosslinking agent may be in the range of 100: 1 to 1: 1.

Optionally, the base body may comprise a main portion, wherein the coating layer is provided over at least portion of an outer surface of the main portion.

Optionally, the coating layer may cover the entire outer surface of the main portion.

Optionally, the coating layer may have a plurality of coating portions arranged along an axis of the main portion with intervals.

Optionally, the coating layer may comprise a helix around an axis of the main portion.

Optionally, the coating layer may be a mesh structure.

Optionally, the coating layer may further comprise a drug.

Optionally, the base body may comprise a balloon body or a stent.

To the above end, the present invention also provides a medical system comprising the medical device as defined above and a catheter body, wherein the base body is sleeved over an outer circumferential surface of a distal end of the catheter body.

Optionally, the crosslinking agent may comprise the photosensitive crosslinker, wherein the medical system further comprises a fiber optic assembly at least partially inserted within the catheter body, the fiber optic assembly comprising a light-emitting member extending to a distal end of the catheter body and being in positional correspondence with the base body, the distal end of the catheter body configured to allow light emitted from the light-emitting member to be irradiated onto the base body.

Optionally, transmittance of the distal end of the catheter body may be greater than 90%.

To the above end, the present invention also provides a method of preparation of the medical device as defined above. The method comprises the steps of:
providing the base body and a coating solution containing the protein and the crosslinking agent;
applying the coating solution onto the at least portion of the outer surface of the base body; and
forming the coating layer by drying the coating solution.

The device, system and method of the present invention provide the following advantages over the prior art:
The medical device includes a radially expandable and collapsible base body and a coating layer disposed over at least portion of an outer surface of the base body. The coating layer contains a protein and a crosslinking agent. The base body is, for example, a balloon or stent, which can be delivered into an organ of a patient, such as a blood vessel. The protein in the coating layer can be transferred onto the wall of the blood vessel and undergo cross-linking reactions, as an exogenous protein, with collagen and elastin on the vessel wall in the presence of the crosslinking agent to form a cross-linked structure in the form of a mesh. The cross-linked structure comprises good mechanical properties and can serve as an in-situ "protein micro-scaffold" to support the blood vessel to maintain the size of the blood vessel after it has been dilated. This prevents the blood vessel from experiencing significant recoil upon withdrawal of the base body, lowering the risk of restenosis in the blood vessel.

### BRIEF DESCRIPTION OF THE DRAWINGS

The accompanying drawings are provided to facilitate a better understanding of the present invention and do not unduly limit the scope thereof in any sense, in which:
Fig. 1 shows a schematic diagram of a medical balloon catheter according to an embodiment of the present invention;
Fig. 2 is a schematic partial view of a medical balloon catheter according to an embodiment of the present invention, showing a coating layer covering an entire outer surface of a main portion of a balloon body;
Fig. 3 is a schematic partial view of a medical balloon catheter according to an embodiment of the present invention, showing a coating layer consisting of multiple coating portions spaced along an axis of a main portion;
Fig. 4 is a schematic partial view of a medical balloon catheter according to an embodiment of the present invention, showing a coating layer which is a mesh structure;
Fig. 5 schematically illustrates a medical balloon catheter according to an embodiment of the present invention, including a balloon body provided thereon with a coating layer;
Fig. 6 shows a cross-sectional view taken along A-A of the medical balloon catheter of Fig. 1;
Fig. 7 is a schematic partial view of a fiber optic assembly in a medical balloon catheter according to an embodiment of the present invention;

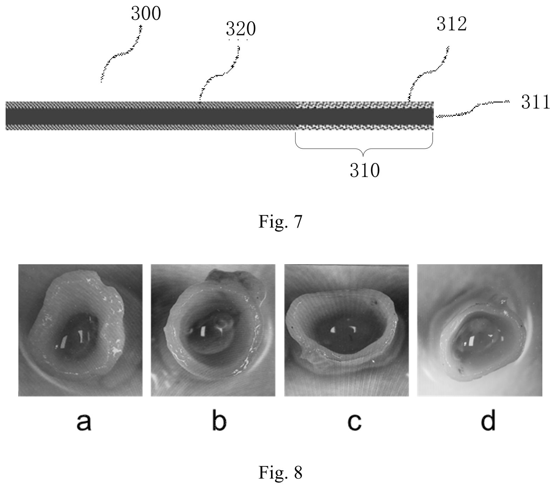

Fig. 8 schematically illustrates a cross-sectional size comparison made of blood vessels, which have undergone treatment with respective medical balloon catheters prepared in accordance with Comparative Example 1, Comparative Example 2, Example 1 of the present application and Comparative Example 3, in which: (a) represents a blood vessel which has undergone treatment with the medical balloon catheter from Comparative Example 1; (b) represents a blood vessel which has undergone treatment with the medical balloon catheter from Comparative Example 2; (c) represents a vessel represents a blood vessel which has undergone treatment with the medical balloon catheter from Example 1 of the present application; (d) represents a blood vessel which has undergone treatment with the medical balloon catheter from Comparative Example 3; and

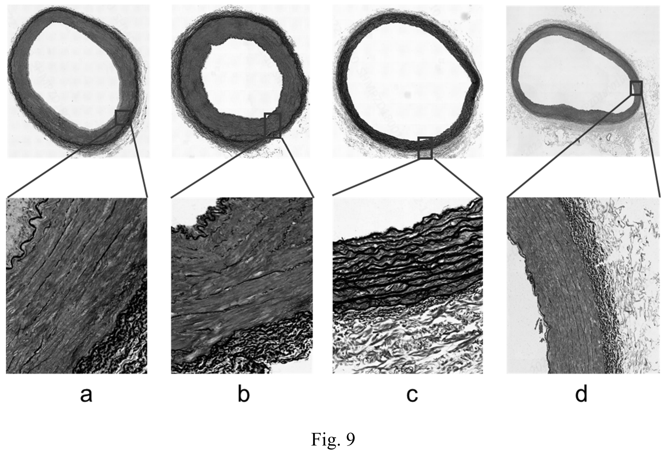

Fig. 9 shows photographs of cross-sections of the blood vessels, which have been stained after being treated with the respective medical balloon catheters prepared in accordance with Comparative Example 1 , Comparative Example 2, Example 1 of the present application and Comparative Example 3, in which: (a) represents a blood vessel which has undergone treatment with the medical balloon catheter from Comparative Example 1; (b) represents a blood vessel which has undergone treatment with the medical balloon catheter from Comparative Example 2; (c) represents a vessel represents a blood vessel which has undergone treatment with the medical balloon catheter from Example 1 of the present application; (d) represents a blood vessel which has undergone treatment with the medical balloon catheter from Comparative Example 3 .

### Reference Numerals

100-catheter body; 110-inner tube; 111-guide wire lumen; 120-outer tube; 200-medical balloon; 210-balloon body; 211-main portion; 212-connecting portion; 220-coating layer; 221-coating portion; 300-fiber optic assembly; 310-light-emitting member; 311-fiber optic core; 312-scattering layer; 320-protective layer; 400-connector; 410-first interface; 420-second interface; 500-guide head; 600-radiopaque element;
10 nozzle; 20 mandrel.

### DETAILED DESCRIPTION

Embodiments of the present invention will be described below by way of specific examples. Based on the disclosure and teachings provided herein, a person of ordinary skill in the art will readily realize other advantages and benefits provided by the present invention. The present invention may also be otherwise embodied or applied through different embodiments, and various modifications or changes may be made to the details disclosed herein from different points of view or for different applications, without departing from the spirit of the present invention. It should be noted that the accompanying drawings are provided herein merely to schematically illustrate the basic concept of the present invention. Accordingly, they only show components relating to the present invention but not necessarily depict all the components as well as their real shapes and dimensions in practical implementations. In practice, the configurations, number and relative scales of the components may vary arbitrarily and their arrangements may be more complicated.

In the following, each of the embodiments is described as having one or more technical features. However, this does not mean that the present invention must be practiced necessarily with all such technical features, or separately with some or all the technical features in any of the embodiments. In other words, as long as the present invention can be put into practice, a person skilled in the art may choose some or all of the technical features in any of the embodiments or combine some or all of the technical features in different embodiments based on the teachings herein and depending on relevant design specifications or the requirements of practical applications. In this way, the present invention can be carried out more flexibly.

As used herein, the terms "proximal end" and "distal end" may be used to describe relative orientations, positions and directions of components of a medical device or actions taken thereon, as viewed by a surgeon operating the medical device. While not intended to be limiting, a "proximal end" usually refers to an end closer to the operator, while a "distal end" usually refers to an end that enters the body of a patient first, during normal use of the medical device.

It is an object of the present invention to provide a medical system including a medical device having a base body, which can be introduced into a patient's body and then expanded, forming an in-situ "protein micro-scaffold" on a blood vessel wall. Supported by the protein micro-scaffold, the blood vessel can be maintained at a dilated size as much as possible without significant recoil, preventing restenosis of the blood vessel that may otherwise occur.

Objects, advantages and features of the present invention will become more apparent upon reading the following more detailed description of the present invention with reference to the accompanying drawings. Note that the figures are provided in a very simplified form not necessarily drawn to exact scale and for the only purpose of facilitating easy and clear description of the embodiments. Throughout these drawings, same numerals indicate same elements.

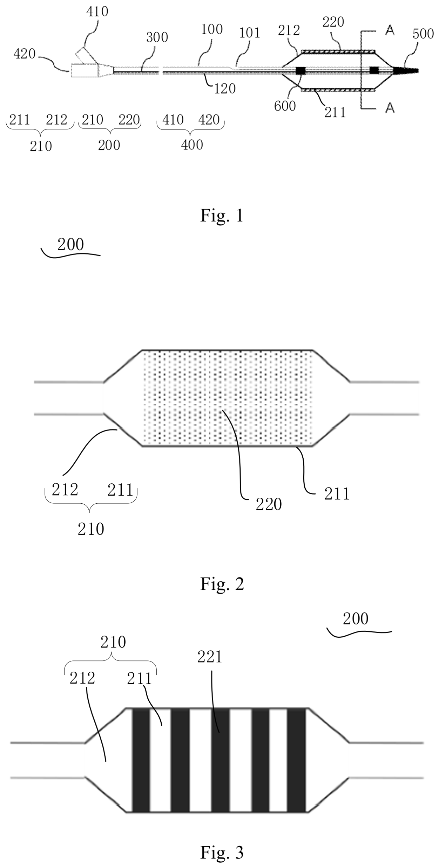

The medical system includes a medical device having a radially expandable and collapsible base body. As a typical example, the base body may be a balloon body 210 (as shown in Fig. 1) or stent. In the case of the base body being provided as a balloon body, the medical device may be a medical balloon, and the medical system may be a medical balloon catheter. The following description is set forth in the context of the medical system being a medical balloon catheter, as an example. Those skilled in the art will recognize that the following description also applies, mutatis mutandis, to scenarios where the base body is a stent or other medical device capable of bearing a coating layer.

Fig. 1 is a schematic diagram showing the medical balloon catheter. As shown in Fig. 1, the medical balloon catheter includes a catheter body 100 and a medical balloon 200. The catheter body 100 comprises an inflation fluid passage. The medical balloon 200 includes a balloon body 210 and a coating layer 220, and the balloon body 210 is sleeved over an outer circumferential surface of the catheter body 100 around a distal end thereof. The balloon body 210 comprises an inflation lumen in communication with the inflation fluid passage. The coating layer 220 is provided on at least a portion of an outer surface of the balloon body 210 and contains a protein and a crosslinking agent. The protein can be cross-linked with proteins on a blood vessel wall under the action of the crosslinking agent.

The medical balloon catheter can be used for the treatment of atherosclerosis. In practical use, the medical balloon 200 may be delivered into a diseased blood vessel in a patient, and an inflation fluid may be introduced through the inflation fluid passage into the inflation lumen of the balloon body 210 to dilate the balloon body 210, such that the coating layer 220 on the outer surface of the balloon body 210 is brought into close contact with the wall of the blood vessel. As an exogenous protein, the protein in the coating layer 220 can be released and transferred onto the blood vessel wall within a short period of time ranging from about 30 s to 60 s, creating a high protein concentration over a portion of the vessel wall where it contacts the coating layer 220. Those skilled in the art will know that the wall of a blood vessel consists of the intima, media, and adventitia. The intima consists of a lining of squamous endothelial cells and the media is separated from the intima by the internal elastic lamina, and the media consists of alternating layers of elastic lamellae, elastic fibers and smooth muscle cells. The adventitia is composed of collagen and elastic fibers. Thus, a vascular wall contains elastin and extracellular matrix proteins. It has been known that proteins are able to cross-link to form a stretchable mesh with certain mechanical properties. According to embodiments of the present invention, the exogenous protein from the coating layer 220 can be fully cross-linked with elastin and extracellular matrix proteins on the vessel wall under the action of the crosslinking agent, thereby forming an in-situ "protein micro-scaffold" in the form of a cross-linked mesh with good mechanical properties, which provides support to the blood vessel that has been dilated by the medical balloon 200. In this way, the blood vessel is maintained at the dilated size to a certain extent and will not recoil even after the balloon body 210 is withdrawn. Thus, the luminal gain is retained, and the occurrence of restenosis can be reduced or even eliminated, allowing remodeling of the blood vessel. Besides these, the protein micro-scaffold will not degrade and addresses the problems that may arise from residuals of a foreign implant.

According to embodiments of the present invention, additionally introducing the exogenous protein through the coating layer 220 can create an increased protein concentration over the portion of the vessel wall where it contacts the coating layer 220, forming more cross-linked products. This can enhance the mechanical properties of the resulting protein micro-scaffold and effectively avoid its inability to provide sufficient support to the vessel wall due to absence of or inadequate cross-linking reactions due to low presence of proteins on the vessel wall or over the region thereof.

In addition, as an artifact, the coating layer 220 can be made to a predetermined desired shape on the outer surface of the balloon body 210 during production phase, which enables a protein concentration over a vessel wall portion of interest to be modified as a result of the medical balloon 200 being delivered into the blood vessel. Typically, the concentration of protein is higher in the region of the vessel wall that is in contact with coating layer 220, and the concentration of protein is lower in the region of the vessel wall that is not in contact with coating layer 220.

Specifically, as shown in Fig. 1, the balloon body 210 includes a main portion 211 and two connecting portions 212 joined to respective axial ends of the main portion 211. The coating layer 220 is sleeved over at least portion of an outer surface of the main portion 211. It should be noted that in the case of the base body being implemented as a stent, it includes only the main portion, but not the connecting portions.

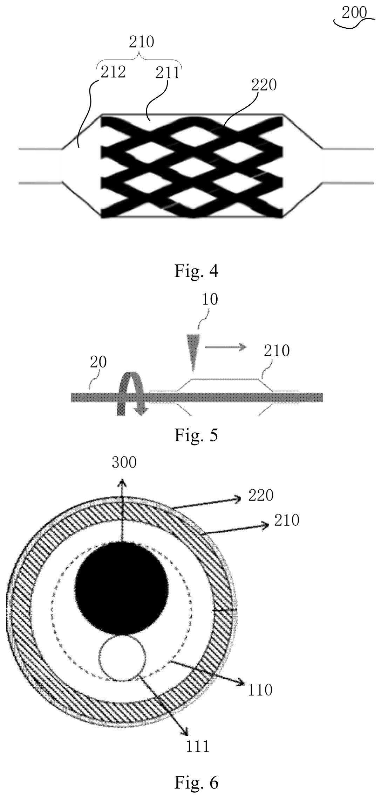

More specifically, in some embodiments, as shown in Fig. 2, the coating layer 220 covers the entire outer surface of the main portion 211. Accordingly, a portion of the vessel wall corresponding to the main portion 211 can be entirely brought into contact with the coating layer 220 and receive the exogenous protein from the coating layer 220, gaining an increase in protein concentration. In some alternative embodiments, as shown in Fig. 3, the coating layer 220 includes multiple coating portions 221 spaced along an axis of the main portion 211. In these embodiments, portions of the vessel wall brought into contact with the respective coating portions 221 receive the exogenous protein from the coating layer 220, thereby comprising a higher protein concentration, while portions of the vessel wall corresponding to gaps between adjacent coating portions 221 comprise a lower protein concentration due to absence of contact with the coating layer 220. In some other alternative embodiments, as shown in Fig. 4, the coating layer 220 is a mesh, and portions of the vessel wall corresponding to g openings of the mesh will not be brought into contact with the coating layer 220 and therefore retain at a relatively low protein concentration. In yet some alternative embodiments, the coating layer 220 is a helix structure extending around the main portion 211, with portions of the vessel wall corresponding to gaps between adjacent turns of the helix structure retaining a low protein concentration due to absence of contact with the coating layer 220. These implementations of the coating layer 220 on different locations of the main portion 211 may form micro-scaffolds with distinct mechanical properties.

The coating layer 220 may be formed by drying a coating solution applied to the main portion 211 of the balloon body 210. The solution contains the protein and the crosslinking agent. The protein and the crosslinking agent may be dissolved in a solvent with the aid of ultrasound. The solvent may include, without limitation, at least one of ethanol, ethyl acetate, acetone, tetrahydrofuran (THF) and water.

According to embodiments of the present invention, the coating solution may be applied to the balloon body 210 using any suitable approach, as desired. In a typical embodiment, single-nozzle printing may be employed, such as ultrasonic spray printing. Specifically, as shown in Fig. 5, before spraying, the balloon body 210 may be sleeved over a mandrel 20 arranged under a nozzle 10, and the mandrel 20 may be then rotated to cause synchronous rotation of the balloon body 210. The nozzle 10 is translated along an axis of the balloon body 210, the coating solution is jetted from the nozzle, thereby achieving spray printing. It is understood that the entire spray printing process may be controlled by a computer to cause the rotation of the mandrel 20 and the translation and jetting of and from the nozzle 10 to occur at pre-programmed speeds so that the coating solution is sprayed onto the main portion 211 along a predetermined path. Consequently, the coating solution may be applied to the entire outer surface of the main portion 211, or the coating solution forms multiple rings spaced along the axis of the main portion 211 on the outer surface of the main portion 211, or the coating solution forms a grid structure or a helix structure on the main portion 211. In other words, according to practical applications and different program settings, the coating layer 220 may be formed of different patterns on the main portion 211, which result in the formation of the micro-scaffold with different mechanical properties on the vessel wall.

Of course, in other implementations, the coating solution may also be applied to the main portion 211 of the balloon body 210 simply by dip coating.

According to embodiments of the present invention, the protein may include, without limitation, at least one of humanized elastin, humanized collagen, human serum albumin, fibroin and proteoglycan. Fibroin is naturally-occurring proteinaceous macromolecules exhibiting excellent biosafety and biodegradability, an ordered molecular structure and good mechanical properties. When cross-linked into a mesh, fibroin is better oriented, imparting better mechanical properties to the cross-linked structure. For these reasons, according to embodiments of the present invention, the protein is preferred to include fibroin.

Optionally, the crosslinking agent may include a photosensitive crosslinker, which crosslinks protein molecules upon exposure to light. In this case, the crosslinking agent provides both crosslinking properties and photosensitivity. It is activated to produce radicals, when exposed to light, triggering crosslinking.

Alternatively, the crosslinking agent may include a non-photosensitive crosslinker, which crosslinks protein molecules without needing exposure to light. According to embodiments of the present invention, the protein and the crosslinking agent are desired to undergo a cross-linking reaction on the vessel wall, but not under in vitro conditions. To this end, the coating layer 220 is preferred to further contain an isolator for isolating the protein from the non-photosensitive crosslinker, preventing them from experiencing a cross-linking reaction under in vitro conditions.

In an optional implementation, the coating solution contains the protein, the isolator and the crosslinking agent, with the isolator surface-coating the protein. In this implementation, the isolator is present substantially in the form of microspheres or microcapsules effectively encapsulating the protein. Such microspheres and microcapsules may be prepared using conventional techniques. For example, such microspheres may be prepared through emulsification followed by solvent volatilization, and such microcapsules may be prepared using in-situ polymerization. Alternative, in another optional implementation, the coating layer 220 consists of multiple layers, for example, three layers, namely, a first layer, a second layer and a third layer. The first layer contains the protein, but not the non-photosensitive crosslinker. The second layer contains the isolator, and the third layer contains the non-photosensitive crosslinker but not the protein. The second layer is situated between the first and third layers. In this implementation, the individual layers may be separately formed by respective solutions, resulting in greater ease of implementation. It should be noted that the isolator, after being delivered into the blood vessel, is decomposed, dissolved or degraded in body fluids, allowing for contact and cross-linking between the protein and the non-photosensitive crosslinker. Optionally, the isolator may include, without limitation, a sustained-release material such as a phospholipid, starch, polylactic acid, chitosan, etc.

It should also be noted that, in some embodiments, the crosslinking agent may contain both photosensitive and non-photosensitive crosslinkers.

It is understood that the crosslinking agent may be a physical or chemical crosslinker. The physical crosslinker may include at least one of tannic acid, pentagalloylglucose, a gallate, gallic acid, ellagic acid, proanthocyanidin, anthocyanin, quercetin, lignin (yellow), catechin and epicatechin. The chemical crosslinker may include at least one of glutaraldehyde, a polyepoxide, genipin, methylene blue, methylene green, rose Bengal, riboflavin, proflavin, fluorescein, eosin, horseradish catalase, 4-amino-1,8-naphthalimide, 2,2'-((ethane-1,2-diylbis(oxy))bis(ethane-2,1-diyl))bis(6-((2-(2-(2-aminoethoxy)ethoxy)ethyl)amino)-1H-benzo[de]isoquinoline -1,3(2H)-dione) or salt thereof, ammonium persulfate, tris(bipyridine)ruthenium(II) chloride, camphorquinone and curcumin.

Typically, the physical crosslinker is the non-photosensitive crosslinker. Among the chemical crosslinker, some are non-photosensitive, and the others are photosensitive. Specifically, the photosensitive examples of the chemical crosslinker are methylene blue, methylene green, rose Bengal, riboflavin, proflavin, fluorescein, eosin, 4-amino-1,8-naphthalimide and 2,2'-((ethane-1,2-diylbis(oxy))bis(ethane-2,1-diyl))bis(6-((2-(2-(2-aminoethoxy)ethoxy)ethyl)amino)-1H-benzo[de]isoquinoline -1,3(2H)-dione).

Those skilled in the art will recognize that riboflavin, also known as vitamin B2, can reduce inflammation and improve cardiac blood supply. According to preferred embodiments of the present invention, the crosslinking agent includes riboflavin.

In the coating solution, the protein and the crosslinking agent may be present at a ratio by weight, as desired. This ratio is typically in the range of 100: 1 to 1: 1, such as for example, 100: 1, 90: 1, 80: 1, 70: 1, 65: 1, 55: 1, 40: 1, 30: 1, 20: 1, 10: 1, 5: 1, 2: 1, 1: 1 or the like.

According to more preferred embodiments, the coating layer 220 further contains a drug. The drug may be of any type, as desired. For example, it may be an antiproliferative, anti-inflammatory, antiphlogistic, anti-hyperplastic, antibacterial, antineoplastic, anti-mitotic, cytostatic, cytotoxic, anti-osteoporotic, anti-angiogenic, anti-restenotic, antimicrotubular, anti-metastatic, anti-thrombotic or other drug. Particular examples of the drug, may include, but are not limited to, dexamethasone, prednisolone, corticosterone, budesonide, estrogen, sulfasalazine and mesalamine, acemetacin, aescin, aminopterin, antimycoin, arsenic trioxide, aristolochic acids, aspirin, berberine, bilobol, endostatin, angiostatin, angiopeptin, monoclonal antibodies capable of blocking proliferation of smooth muscle cells, levofloxacin, paclitaxel, docetaxel, hydroxycamptothecin, vinblastine, vincristine, doxorubicin, 5-fluorouracil, cisplatin, antibiotics with inhibitory activity against thymidine-kinase (in particular, actinomycin-D), hormones, antibodies for cancer therapy, bisphosphonates, selective estrogen receptor modulators, strontium ranelate, cyclosporine A, cyclosporine C, brefeldin A, vascular endothelial growth factors, angiogenin, recombinant proteins, stem cells, rapamycin and derivatives thereof. The rapamycin derivatives may include, but are not limited to, zotarolimus, everolimus, biolimus, 7-O-desmethyl rapamycin, temsirolimus and deforolimus.

In order to enable use of the medical balloon catheter for the treatment of atherosclerosis, the coating layer 220 may further contain a drug capable of preventing or restraining coronary microcirculatory dysfunction. Such a drug may include, without limitation, at least one of statins (e.g., ACEI/ARB), ranolazine, metoprolol, adenosine, nitrates, sodium nitroprusside, verapamil, nicorandil and alprostadil.

As noted above, the crosslinking agent in the coating layer 220 may include a photosensitive crosslinker, of which activation by light is necessary. In this case, as shown in Figs. 1, 6 and 7, the medical balloon catheter may further include a fiber optic assembly 300 including a light-emitting member 310 disposed at a distal end thereof. The fiber optic assembly 300 may be at least partially inserted into the catheter body 100 and the light-emitting member 310 extends to a location around a distal end of the catheter body 100, and the light-emitting member 310 is in positional correspondence with the balloon body 210. The distal end of the catheter body 100 is configured to allow light from the light-emitting member 310 to be irradiated onto the balloon body 210, thus activating the photosensitive crosslinker. Radicals produced by the activated crosslinker can trigger cross-linking of the protein. An axial length of the light-emitting member 310 may be selected from the range of 1 mm to 50 mm, as desired.

According to preferred embodiments of the present invention, the fiber optic assembly 300 is cylindrical light-diffusing fiber, and is able to laterally emit diffuse light. In a particular embodiment, the fiber optic assembly 300 includes a fiber optic core 311, a distal end of the fiber optic core 311 extends to the distal end of the catheter body 100, and it is in positional correspondence with the balloon body210. The fiber optic assembly 300 further includes a scattering layer 312 disposed over at least the light-emitting member 310 and uniformly attached to an outer circumferential surface of the fiber optic core 311. The scattering layer 312 contains a matrix material and scattering particles uniformly dispersed throughout the matrix material. The scattering particles can scatter light from the fiber optic core 311 to provide circumferentially uniform diffuse light.

According to embodiments of the present invention, the fiber optic core 311 has a diameter in the range of from 0.05 mm to 1 mm, and the scattering layer 312 has a thickness of from 0.1 mm to 0.5 mm. The matrix material may include, without limitation, rubber, and the scattering particles may include, without limitation, titanium dioxide nanoparticles. It should be noted that, in the case of the scattering layer 312 being disposed over only the light-emitting member 310, the fiber optic assembly 300 may further include a protective layer 320, which is attached to an outer circumferential surface of the fiber optic core 311 on a proximal side of the scattering layer 312.

Further, referring to Fig. 6, the catheter body 100 includes an inner tube 110 and an outer tube 120. The inner tube 110 is partially inserted through the outer tube 120 and its distal end protrudes beyond a distal end of the outer tube 120. The inflation fluid passage is formed between an inner surface of the outer tube 120 and an outer surface of the inner tube 110. A distal end of the outer tube 120 is sealingly attached to a proximal one of the connecting portions 212 in the balloon body 210 so that the inflation fluid passage is brought into communication with the inflation lumen of the balloon body 210. The inner tube 110 comprises a fiber optic lumen (not shown), and at least part of the fiber optic assembly 300 is inserted within the fiber optic lumen of the inner tube 110. A distal portion of the inner tube 110 is configured to allow transmission of light therethrough. Optionally, the entire inner tube 110 may be made of a transparent polyethylene material, and its transmittance may be greater than 90%. Thus, light from the light-emitting member 310 can transmit through the inner tube 110.

Additionally, as is conventional, the inner tube 110 also comprises a guide wire lumen 111 axially extending through the inner tube 110 and being parallel to the fiber optic lumen, and a guide wire aperture 101 is formed in a wall of the catheter body 100 around a proximal end and communicates with the guide wire lumen 111. The guide wire aperture 101 is configured to introduce a guide wire. The medical balloon catheter may further include a connector 400 attached to the proximal end of the catheter body 100, which includes a first interface 410 and a second interface 420. The first interface 410 communicates with the inflation fluid passage and is configured for connection with an inflator such as a syringe. The second interface 420 is configured for communication with the inner tube 110 and for passage of a proximal end of the fiber optic assembly 300 therethrough. The medical balloon catheter may further include a guide head 500 and a radiopaque element 600. The guide head 500 is connected to the distal end of the inner tube 110 and is located on a distal side of the medical balloon 200 and is connected to a distal end of the fiber optic assembly 300. Typically, the guide head 500 is a tapered structure and has an inner diameter gradually decreasing in a proximal-to-distal direction. With this arrangement, it can provide guidance to distal advancement of the medical balloon catheter within a blood vessel. The radiopaque element 600 is disposed on the inner tube 110 at a location in positional correspondence with the medical balloon 200. It is visible under X-rays, helping locate the medical balloon 200.

Embodiments of the present invention also provide a method of preparation, suitable for use to prepare a medical device as discussed above. The method includes the steps of:
first providing a radially expandable and collapsible base body (e.g., the balloon body 210) and a coating solution containing a protein and a crosslinking agent;
then spraying the coating solution onto at least portion of an outer surface of the balloon body 210; and
finally, drying the coating solution to form a coating layer 220.

Next, use of the method in Example 1 is described, and medical balloon catheters prepared in accordance with Example 1 and Comparative Examples are compared to demonstrate therapeutic benefits that embodiments of the present invention offer.

In Example 1, riboflavin was used as a crosslinking agent, the fibroin was used as protein, and a weight ratio of fibroin-to-riboflavin was 10: 1.

At first, a coating solution was prepared. Specifically, the riboflavin and fibroin were added to a solvent and dissolved therein with the aid of ultrasound. In this example, the solvent was a mixture of ethyl acetate and acetone at an ethyl acetate-to-acetone weight ratio of 3: 1.

Subsequently, the coating solution was sprayed onto an outer surface of a main portion 211 of the balloon body 210 with the aid of ultrasound, the entire outer surface of the main portion 211 of the balloon body 210 was covered with the coating solution.

The coating solution was dried and cured at room temperature for 24 h, forming a coating layer 220.

In this example, six medical balloons were prepared, designated as No. 1 to No. 6 medical balloons, and the size of the medical balloons are shown in Table 1 below.

The quantities of fibroin and riboflavin in the coating layers 220 of the six medical balloons 200 were determined as shown in Table 1. As calculated, the fibroin was contained at a density of about 0.13 mg/mm², and the crosslinking agent of approximately 0.012 mg/mm², in the coating layers 220.

The medical balloons 200 were assembled with respective catheter bodies 100 and respective fiber optic assemblies 300 into respective medical balloon catheters, which were then subjected to performance tests.

Samples of a blood vessel of interest were then provided, which was the porcine right iliac artery, with surrounding surface tissue being removed. Each sample was 1 cm long and had a vascular wall thickness of 2 mm and an inner diameter of 5 mm.

The medical balloon catheter prepared from the No.1 medical balloon 200 was first tested, in which the medical balloon 200 had been crimped.

The medical balloon 200 was delivered into the vascular sample, and an inflation fluid was introduced into an inflation lumen of the balloon body 210 through a first interface 410 until the balloon body 210 is inflated so that the coating layer 220 was brought into contact with the vessel wall. In this process, the balloon body 210 was dilated to a diameter that was 125% of an initial luminal diameter of the vessel (before it was treated with the medical balloon 200).

The coating layer 220 was then irradiated with laser light of a wavelength of 450 nm emitted at 1 W from a light-emitting member 310 in the fiber optic assembly 300 for 60 s.

After that, the inflation fluid was discharged away, and the medical balloon 200 was removed from the vessel.

Afterwards, the vessel's wall thickness and inner diameter were measured (Table 2), and a photograph of it was taken (Fig. 8(c)).

At last, the vessel was histochemically stained by being immersed in formalin for a predetermined period of time, and another photograph of it was then taken (Fig. 9(c)).

### Comparative Example 1

This comparative example differs from Example 1 in a coating layer 220 containing fibroin, instead of riboflavin. A medical balloon catheter prepared according to this comparative example was used to treat a blood vessel of interest in the same way as described above, and parameters of the blood vessel before and after the treatment are summarized in Table 2. A photograph taken of the blood vessel after it was treated with the medical balloon catheter according to this comparative example and before it was histochemically stained is shown in Fig. 8(a), and another photograph taken of the vessel after it was histochemically stained is shown in Fig. 9(a).

### Comparative Example 2

This comparative example differs from Example 1 in treatment of a blood vessel of interest not involving irradiating a coating layer 220 with a fiber optic assembly 300. A medical balloon catheter according to this comparative example was prepared in the same way as that of Example 1, and parameters of the blood vessel before and after it was treated with the medical balloon catheter are summarized in Table 2. A photograph taken of the blood vessel after it was treated with the medical balloon catheter according to this comparative example and before it was histochemically stained is shown in Fig. 8(b), and another photograph taken of the vessel after it was histochemically stained is shown in Fig. 9(b).

### Comparative Example 3

This comparative example differs from Example 1 in a coating layer 220 containing riboflavin, instead of fibroin. A medical balloon catheter prepared according to this comparative example was used to treat a blood vessel of interest in the same way as described above, and parameters of the blood vessel before and after the treatment are summarized in Table 2. A photograph taken of the blood vessel after it was treated with the medical balloon catheter according to this comparative example and before it was histochemically stained is shown in Fig. 8(d), and another photograph taken of the vessel after it was histochemically stained is shown in Fig. 9(d).

**Table 1**

| | Length (mm) | Inner Diameter (mm) | Fibroin (mg) | Riboflavin (mg) |
|---|---|---|---|---|
| No. 1 | 13 | 3 | 15.1 | 1.4 |
| No. 2 | 23 | 3 | 28.6 | 2.7 |
| No. 3 | 28 | 3 | 33.9 | 3.2 |
| No. 4 | 13 | 4 | 21.2 | 2.1 |
| No. 5 | 23 | 4 | 37.3 | 3.6 |
| No. 6 | 28 | 4 | 41.3 | 4.4 |

**Table 2**

| | Initial Inner Diameter (mm) | Initial Blood Vessel Wall Thickness (mm) | Inner Diameter after Treatment (mm) | Blood Vessel Wall Thickness after Treatment (mm) |
|---|---|---|---|---|
| Example 1 | 5 | 2 | 6 | 1.5 |
| Comparative Example 1 | 6 | 1.5 | 6 | 1.5 |
| Comparative Example 2 | 5 | 2 | 5 | 2 |
| Comparative Example 3 | 5 | 2 | 5.5 | 1.5 |

As can be seen from Figs. 8 and 9, the cross-section of the blood vessel that was treated with the medical balloon catheter of Example 1 of the present invention was considerably darkened, indicating increased presence of proteins. Moreover, the protein fibers were oriented circumferentially around the blood vessel. In contrast, the cross-sections of the blood vessels treated with the medical balloon catheters of Comparative Examples 1 and 2 each had a lighter color, indicating less presence of proteins, and noticeable orientation of protein fibers was not observed. Although the cross-section of the blood vessel treated with the medical balloon catheter of Comparative Example 3 showed a more or less darker color due to increased presence of proteins and a certain degree of orientation of protein fibers, the color was lighter and the degree of orientation was lower, in comparison with Example 1. This demonstrates that during the treatment of the blood vessel by the medical balloon catheter of the present invention, the protein from the coating layer 220 was deposited and oriented on the vessel wall, forming an in-situ "protein micro-scaffold". In addition, as shown in Table 2, the blood vessel treated with the medical balloon catheter of Example 1 of the present invention had a larger inner diameter and smaller wall thickness than the blood vessels treated with those of Comparative Examples 1 and 2, suggesting good support of the "protein micro-scaffold" for the vessel wall. With such support, the vessel wall could be maintained in a dilated configuration, reducing the risk of restenosis in the blood vessel. Compared with Comparative Example 3, the blood vessel treated with the medical balloon catheter of Example 1 of the present invention exhibited a greater increase in inner diameter. This is because the additional protein in the coating layer led to increased protein presence and hence adequate cross-linking, allowing for the successful formation of the "protein micro-scaffold" with sufficiently strong support, thereby avoiding inadequate support of the protein micro-scaffold so as to effectively support the blood vessel wall.

As described above, embodiments of the present invention provide a medical balloon catheter including a medical balloon provided with a coating layer containing a protein which can be cross-linked by the crosslinking agent with proteins on the wall of a blood vessel, forming an in-situ "protein micro-scaffold", the "protein micro-scaffold" utilizes its mechanical properties to support the dilated blood vessel, thereby maintaining the blood vessel in the dilated configuration. Thus, the risk of the blood vessel experiencing restenosis is reduced.

Although the present invention has been disclosed hereinabove, it is not limited to the above disclosure. Those skilled in the art can make various changes and modifications to the invention without departing from the spirit and scope thereof. Accordingly, it is intended that any and all such changes and modifications also fall within the scope of the present invention as defined by the appended claims and equivalents thereof.

## Claims

1. A medical device, comprising a base body and a coating layer, wherein the base body is radially expandable and collapsible, wherein the coating layer is disposed over at least portion of an outer surface of the base body, and wherein the coating layer comprises a protein and a crosslinking agent.

2. The medical device according to claim 1, wherein the crosslinking agent comprises a photosensitive crosslinker, and wherein when exposed to light, the photosensitive crosslinker undergoes a cross-linking reaction with the protein.

3. The medical device according to claim 2, wherein the photosensitive crosslinker comprises at least one of methylene blue, methylene green, rose Bengal, riboflavin, proflavin, fluorescein, eosin, 4-amino-1,8-naphthalimide, 2,2'-((ethane-1,2-diylbis(oxy))bis(ethane-2,1-diyl))bis(6-((2-(2-(2-aminoethoxy)ethoxy)ethyl)amino)-1H-benzo[de]isoquinoline -1,3(2H)-dione) and salts thereof.

4. The medical device according to claim 1, wherein the crosslinking agent comprises a non-photosensitive crosslinker, wherein the coating layer further comprises an isolator that isolates the protein from the non-photosensitive crosslinker, thereby preventing the protein from undergoing a cross-linking reaction with the non-photosensitive crosslinker.

5. The medical device according to claim 4, wherein the non-photosensitive crosslinker comprises a physical crosslinker and/or a chemical crosslinker, wherein the chemical crosslinker comprises at least one of glutaraldehyde, polyepoxide, genipin, horseradish catalase, ammonium persulfate, tris(bipyridine)ruthenium(II) chloride, camphorquinone and curcumin.

6. The medical device according to claim 5, wherein the physical crosslinker comprises at least one of tannic acid, pentagalloylglucose, gallate, gallic acid, ellagic acid, proanthocyanidin, anthocyanin, quercetin, lignin (yellow), catechin and epicatechin.

7. The medical device according to claim 1, wherein the protein comprises at least one of humanized elastin, humanized collagen, human serum albumin, fibroin and proteoglycan.

8. The medical device according to claim 1, wherein a weight ratio of the protein to the crosslinking agent is in a range of 100: 1 to 1: 1.

9. The medical device according to claim 1, wherein the base body comprises a main portion, wherein the coating layer is provided over at least portion of an outer surface of the main portion.

10. The medical device according to claim 9, wherein the coating layer covers an entire outer surface of the main portion.

11. The medical device according to claim 10, wherein the coating layer comprises a plurality of coating portions arranged along an axis of the main portion with intervals.

12. The medical device according to claim 10, wherein the coating layer comprises a helix structure around an axis of the main portion.

13. The medical device according to claim 10, wherein the coating layer comprises a mesh structure.

14. The medical device according to claim 1, wherein the coating layer further comprises a drug.

15. The medical device according to claim 1, wherein the base body comprises a balloon body or a stent.

16. A medical system, comprising the medical device of any one of claims 1 to 15 and a catheter body, wherein the base body is sleeved over an outer circumferential surface of a distal end of the catheter body.

17. The medical system of claim 16,wherein the crosslinking agent comprises the photosensitive crosslinker, wherein the medical system further comprises a fiber optic assembly at least partially inserted within the catheter body, wherein the fiber optic assembly comprises a light-emitting member extending to the distal end of the catheter body and is in positional correspondence with the base body, wherein the distal end of the catheter body is configured to allow light emitted from the light-emitting member to be irradiated onto the base body.

18. The medical system of claim 17, wherein a transmittance of the distal end of the catheter body is greater than 90%.

19. A method of manufacture of the medical device of any one of claims 1 to 15, comprising the steps of:
providing the base body that is radially expandable and collapsible and a coating solution containing the protein and the crosslinking agent;
applying the coating solution onto the at least portion of the outer surface of the base body; and
forming the coating layer by drying the coating solution.
